# EUROPEAN PATENT SPECIFICATION

(11) **EP 2 423 202 B1**
(45) Date of publication and mention of the grant of the patent: **16.01.2013**
(21) Application number: 11179518.3
(22) Date of filing: 31.08.2011
(51) Int. Cl.: C07D 251/24, C07D 403/04, C07D 413/04, C08K 5/3492

(54) **Triazine-based compound and ultraviolet absorber**
Triazin-basierte Verbindung und UV-Aufnahmevorrichtung
Composé à base de triazine et absorbeur d'ultraviolets

(30) Priority: 31.08.2010 JP 2010195223; 29.08.2011 JP 2011186607
(43) Date of publication of application: 29.02.2012
(73) Proprietor: Fujifilm Corporation, Minato-ku Tokyo (JP)
(72) Inventor: Amasaki, Ichiro, Kanagawa (JP); Kimura, Keizo, Kanagawa (JP); Tsumura, Kyosuke, Kanagawa (JP)
(74) Representative: HOFFMANN EITLE

(56) References cited:
- WO-A1-97/36880

## Description

### BACKGROUND OF THE INVENTION

### 1. Field of the Invention

The present invention relates to a triazine-based compound and an ultraviolet absorber.

### 2. Description of the Related Art

Conventionally, an ultraviolet absorber includes, for example, an inorganic ultraviolet absorber and an organic ultraviolet absorber. The inorganic ultraviolet absorber (see, for example, JP-A-5-339033 (the term "JP-A" as used herein means an "unexamined published Japanese patent application"), JP-A-5-345639, JP-A-6-56466) is excellent in the durability such as weather resistance and heat resistance but on the other hand, the degree of freedom in selection is disadvantageously small, because the absorption wavelength is determined by the band gap of the compound. In particular, an absorber having excellent absorption in the short wavelength ultraviolet region and being excellent in transparency is not known.

In contrast, the organic ultraviolet absorber has high degree of freedom in structural design of the absorber and therefore, absorbers having various absorption wavelengths can be obtained by devising the absorber structure.

Heretofore, various organic ultraviolet absorbers have been studied.

For example, a triazine-based ultraviolet absorber is disclosed in JP-T-2002-524452 (the term "JP-T" as used herein means a published Japanese translation of a PCT patent application), Japanese Patent No. 3,965,631, International Publication No. 97/36880.

JP-T-2002-524452 describes a trisaryl-s-triazine having a hydroxy group in the ortho and para positions with respect to the triazine ring.

Japanese Patent No. 3,965,631 describes a trisaryl-s-triazine having a hydroxy group in the ortho position with respect to the triazine ring and having an alkoxy group in the para position with respect to the triazine ring.

International Publication No. 97/36880 describes a trisaryl-s-triazine having a hydroxy group in the ortho position with respect to the triazine ring and having an amido group in the para position with respect to the triazine ring.

Also, JP-A-2001-277720 describes an amido group-containing trisaryl-s-triazine as a dye compound for use in an optical information recording medium.

### Summary of the Invention

However, out of conventional ultraviolet absorbers, those having a maximum absorption wavelength in the short wavelength ultraviolet region in the vicinity of 300 to 320 nm are poor in the light resistance, and the ultraviolet-blocking effect is reduced with the passing of time.

Accordingly, a compound usable as an ultraviolet absorber exhibiting a blocking effect down to the short wavelength ultraviolet region in the vicinity of 300 to 320 nm and having more excellent light resistance than ever is demanded.

Also, an ultraviolet absorber causing little tinting while having the above-described effects is demanded.

An object of the present invention is to provide a compound useful as an ultraviolet absorber causing little tinting and exhibiting an ultraviolet-blocking effect even in the short wavelength ultraviolet region in the vicinity of 300 to 320 nm and having excellent light resistance. Another object of the present invention is to provide an ultraviolet absorber using the compound.

As a result of detailed studies on a triazine-based compound, the present inventors have found that the above-described problems can be solved by a compound of specific structure having an acylamido group or a sulfonamido group in a specific position (ortho position with respect to a triazine ring) of a 2,4,6-triphenyl-(1,3,5)triazine skeleton. More specifically, it has been found that although conventional triazine-based ultraviolet absorbers have a hydroxyl group in the ortho position with respect to the triazine ring, when the hydroxyl group is changed to an acylamido group or a sulfonamido group, in particular, HOMO is stabilized and in turn, the absorption band (λmax: near 340 nm) on the long wavelength side out of two absorption bands characteristic of conventional triazine-based ultraviolet absorbers can be shifted to the short wavelength side, as a result, the absorber can exhibit a high ultraviolet-blocking effect in the short wavelength ultraviolet region in the vicinity of 300 to 320 nm and be reduced in tinting. The present invention has been accomplished based on this finding.

The objects of the present invention are attained by the following techniques.
[1] A compound represented by the following formula (1): 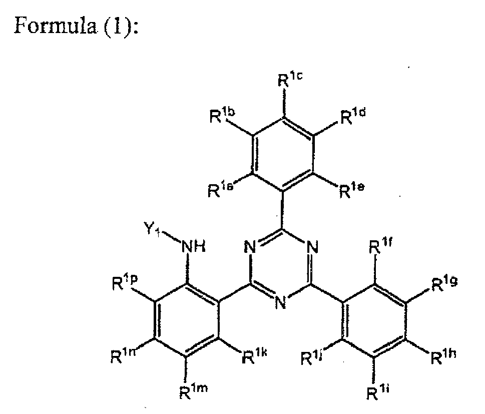 wherein each of R^{1b}, R^{1c}, R^{1d}, R^{1g}, R^{1h}, R¹ⁱ, R^{1k}, R^{1m}, R¹ⁿ and R^{1p} independently represents a hydrogen atom or a monovalent substituent, the monovalent substituents may combine with each other to form a ring, provided that at least one of R^{1c} and R^{1h} represents a hydrogen atom or a substituent having a positive σp value of the Hammett's rule, each of R^{1a}, R^{1e}, R^{1f} and R^{1j} independently represents a hydrogen atom or a monovalent substituent excluding -NHY₁ and -OH, Y₁ represents -COR^{1q} or -SO₂R^{1r}, and each of R^{1q} and R^{1r} independently represents a monovalent substituent.
[2] The compound as described in [1], wherein the compound represented by formula (1) is a compound represented by the following formula (2): 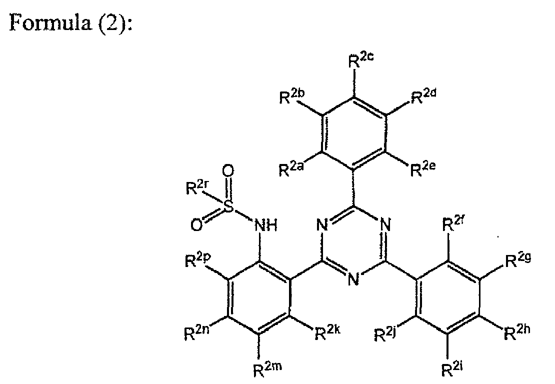 wherein R^{2b}, R^{2c}, R^{2d}, R^{2g}, R^{2h}, R²ⁱ, R^{2k}, R^{2m}, R²ⁿ and R^{2p} have the same meanings as R^{1b} R^{1c}, R^{1d}, R^{1g}, R^{1h}, R¹ⁱ, R^{1k}, R^{1m}, R¹ⁿ and R^{1p} in formula (1), respectively; R^{2a}, R^{2e} R^{2f} and R^{2j} have the same meanings as R^{1a}, R^{1e}, R^{1f} and R^{1j} in formula (1), respectively; and R^{2r} have the same meaning as R^{1r} in formula (1).
[3] The compound as described in [1], wherein R^{1q} represents an alkyl group, a perfluoroalkyl group, an amino group, an aryl group or an alkoxy group.
[4] The compound as described in [1], wherein R^{1r} represents an alkyl group, a perfluoroalkyl group or an aryl group.
[5] The compound as described in [1] or [3], wherein R^{1q} represents an alkyl group or a perfluoroalkyl group.
[6] The compound as described in [1] or [4], wherein R^{1r} represents an alkyl group or a perfluoroalkyl group.
[7] The compound as described in any one of [1] and [3] to [6], wherein R^{1c} is a hydrogen atom, an alkyl group, an alkoxy group, an alkylthio group, an aryl group, a heterocyclic group or a nitro group.
[8] The compound as described in any one of [1] and [3] to [7], wherein R¹ⁿ is a hydrogen atom, an alkoxy group, an amino group, an alkyl group, a sulfonamido group or an aryl group.
[9] The compound as described in any one of [1] and [3] to [7], wherein R¹ⁿ is a hydrogen atom or an alkoxy group.
[10] The compound as described in any one of [1] and [3] to [9], wherein each of R^{1b}, R^{1d}, R^{1g}, R^{1h}, R¹ⁱ, R^{1k}, R^{1m} and R^{1p} is independently a hydrogen atom, an alkoxy group, an aryl group, a cyano group, an alkoxycarbonyl group, an acylamido group, an alkenyl group, a sulfamoyl group, an acyl group or a hydroxy group.
[11] The compound as described in any one of [1] and [3] to [9], wherein each of R^{1b}, R^{1d}, R^{1g}, R^{1h}, R¹ⁱ, R^{1k}, R^{1m} and R^{1p} is a hydrogen atom.
[12] The compound as described in any one of [1] and [3] to [11], wherein each of R^{1a}, R^{1e} and R^{1j} is a hydrogen atom.
[13] The compound as described in any one of [1] and [3] to [12], wherein R^{1f} is a hydrogen atom, an acyloxy group, a formyl group, an arylthio group or a halogen atom.
[14] The compound as described in any one of [1] and [3] to [6], wherein at least two of R^{1b}, R^{1c} and R^{1d}, a pair of R^{1h} and R¹ⁱ, or at least two of R^{1m}, R¹ⁿ and R^{1p} are combined to form a ring.
[15] An ultraviolet absorber comprising the compound described in any one of [1] to [14].

According to the present invention, a compound causing little tinting, exhibiting a blocking effect even in the short wavelength ultraviolet region in the vicinity of 300 to 320 nm and having excellent light resistance, and an ultraviolet absorber can be provided.

### DETAILED DESCRIPTION OF THE INVENTION

The present invention is described in detail below.

### [Compound Represented by Formula (1)]

The present invention relates to a compound represented by the following formula (1): 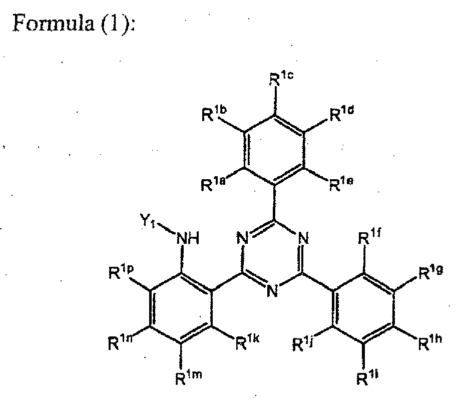 wherein each of R^{1b}, R^{1c}, R^{1d}, R^{1g}, R^{1h}, R¹ⁱ, R^{1k}, R^{1m}, R¹ⁿ and R^{1p} independently represents a hydrogen atom or a monovalent substituent, the monovalent substituents may combine with each other to form a ring, provided that at least one of R^{1c} and R^{1h} represents a hydrogen atom or a substituent having a positive σp value of the Hammett's rule, each of R^{1a}, R^{1e}, R^{1f} and R^{1j} independently represents a hydrogen atom or a monovalent substituent excluding -NHY₁ and -OH, Y₁ represents -COR^{1q} or -SO₂R^{1r}, and each of R^{1q} and R^{1r} independently represents a monovalent substituent.

Examples of the monovalent substituent in formula (1) include substituents selected from the following Substituent Group A:

### (Substituent Group A)

a halogen atom (e.g., fluorine atom, chlorine atom, bromine atom, iodine atom), an alkyl group (preferably an alkyl group having a carbon number of 1 to 20, e.g., methyl group, ethyl group), an aryl group (preferably an aryl group having a carbon number of 6 to 20, e.g., phenyl group, naphthyl group), a cyano group, a carboxyl group, an alkoxycarbonyl group (e.g., methoxycarbonyl group), an aryloxycarbonyl group (e.g., phenoxycarbonyl group), a substituted or unsubstituted carbamoyl group (e.g., carbamoyl group, N-phenylcarbamoyl group, N,N-dimethylcarbamoyl group), an alkylcarbonyl group (e.g., acetyl group), an arylcarbonyl group (e.g., benzoyl group), a nitro group, a substituted or unsubstituted amino group (e.g., amino group, dimethylamino group, aniline group, substituted sulfoamino group), an acylamido group (e.g., acetamido group, ethoxycarbonylamino group), a sulfonamide group (e.g., methanesulfonamido group), an imido group (e.g., succinimido group, phthalimido group), an imino group (e.g., benzylidene amino group), a hydroxy group, an alkoxy group (an alkoxy group having a carbon number of 1 to 20, e.g., methoxy group), an aryloxy group (e.g., phenoxy group), an acyloxy group (e.g., acetoxy group), an alkylsulfonyloxy group (e.g., methanesulfonyloxy group), an arylsulfonyloxy group (e.g., benzenesulfonyloxy group), a sulfo group, a substituted or unsubstituted sulfamoyl group (e.g., sulfamoyl group, N-phenylsulfamoyl group), an alkylthio group (e.g., methylthio group), an arylthio group (e.g., phenylthio group), a thiocyanato group, an alkylsulfonyl group (e.g., methanesulfonyl group), an arylsulfonyl group (e.g., benzenesulfonyl group), a heterocyclic group (a heterocyclic group having a carbon number of 1 to 20, e.g., pyridyl group, pyrazinyl group, morpholino group, azole group), an alkenyl group (preferably an alkenyl group having a carbon number of 2 to 20, e.g., vinyl group, allyl group), an acyl group (preferably an acyl group having a carbon number of 2 to 20, e.g., acetyl group, propionyl group, 2.ethylhexanoyl group), and a formyl group.

The monovalent substituent in formula (1) may be further substituted, and examples of the further substituent include a substituent selected from Substituent Group A.

As for the monovalent substituent in formula (1), when a plurality of substituents are present, they may be the same or different.

As for the monovalent substituent in formula (1), two or more substituents may combine with each other to form a ring.

The ring formed by combining substituents with each other is preferably an aliphatic hydrocarbon ring, an aromatic hydrocarbon ring or an aromatic heterocyclic ring, and examples thereof include a benzene ring, a naphthalene ring, a pyridine ring, a pyrazine ring, a pyrimidine ring, a triazine ring, a pyridazine ring, a pyrrole ring, a pyrazole ring, an imidazole ring, a triazole ring, an oxazole ring, an oxadiazole ring, a thiazole ring, a thiadiazole ring, a furan ring, a thiophene ring, a benzothiophene ring, a selenophene ring, a silole ring, a germole ring, a phosphole ring, a benzoquinone ring, and a cyclohexane ring.

The ring above may further have a substituent, and examples of the substituent include a substituent selected from Substituent Group A. Also, two or more substituents may combine with each other to further form a ring. Examples of the ring are the same as above.

As a preferable embodiment when a ring is formed, in formula (1), at least one embodiment among an embodiment where at least two members out of R^{1a}, R^{1b}, R^{1c}, R^{1d} and R^{1e} combine to form a ring, an embodiment where at least two members out of R^{1f}, R^{1g}, R^{1h}, R¹ⁱ and R^{1j} combine to form a ring, and an embodiment where at least two members out of R^{1k}, R^{1m}, R¹ⁿ and R^{1p} combine to form a ring is preferred.

In formula (1), at least one embodiment among an embodiment where at least two members out of R^{1b}, R^{1c} and R^{1d} combine to form a ring, an embodiment where R^{1h} and R¹ⁱ combine to form a ring, and an embodiment where at least two members out of R^{1m}, R¹ⁿ and R^{1p} combine to form a ring is preferred.

In formula (1), each of R^{1b}, R^{1c}, R^{1d}, R^{1g}, R^{1h}, R¹ⁱ, R^{1k}, R^{1m}, R¹ⁿ and R^{1p} independently represents a hydrogen atom or a monovalent substituent.

In the case where each of R^{1b}, R^{1c}, R^{1d}, R^{1g}, R^{1h}, R¹ⁱ, R^{1k}, R^{1m}, R¹ⁿ and R^{1p} represents a monovalent substituent, the monovalent substituent includes a substituent selected from Substituent Group A.

Each of R^{1b}, R^{1d}, R^{1g}, R^{1h}, R¹ⁱ, R^{1k}, R^{1m} and R^{1p} is preferably a hydrogen atom, an alkoxy group (an alkoxy group having a carbon number of 1 to 20, preferably an alkoxy group having a carbon number of 1 to 10, more preferably a methoxy group or an ethoxy group, still more preferably a methoxy group), an aryl group (preferably an aryl group having a carbon number of 6 to 20, e.g., phenyl group, naphthyl group), a cyano group, an alkoxycarbonyl group, an acylamido group, an alkenyl group (preferably an alkenyl group having a carbon number of 2 to 20, e.g., vinyl group, allyl group), a sulfamoyl group, an acyl group (preferably an acyl group having a carbon number of 2 to 20, e.g., acetyl group, propionyl group, 2-ethylhexanoyl group) or a hydroxy group, more preferably a hydrogen atom, an alkoxy group or an aryl group, and still more preferably a hydrogen atom in view of raising the gram extinction coefficient.

R^{1c} is, in view of raising the gram extinction coefficient, preferably a hydrogen atom, an alkyl group (preferably an alkyl group having a carbon number of 1 to 20, more preferably an alkyl group having a carbon number of 1 to 10, still more preferably a methyl group or an ethyl group, yet still more preferably a methyl group), an alkoxy group (an alkoxy group having a carbon number of 1 to 20, preferably an alkoxy group having a carbon number of 1 to 10, more preferably a methoxy group or an ethoxy group, still more preferably a methoxy group), an alkylthio group (preferably an alkylthio group having a carbon number of 1 to 20, more preferably an alkylthio group having a carbon number of 1 to 10, still more preferably a methylthio group or an ethylthio group, yet still more preferably a methylthio group), an aryl group (preferably an aryl group having a carbon number of 6 to 20, e.g., phenyl group, naphthyl group), a heterocyclic group (preferably a nitrogen-containing aromatic heterocyclic group, e.g., pyridyl group, pyrazinyl group, azole group) or a nitro group, more preferably a hydrogen atom, an alkoxy group or an aryl group, still more preferably a hydrogen atom or an alkoxy group, yet still more preferably a hydrogen atom.

However, at least one of R^{1c} and R^{1h} represents a hydrogen atom or a substituent having a positive σp value of the Hammett's rule.

In the present invention, the values described in Chemical Reviews, 1991, vol. 91, 165-195 are used for the σp value of the Hammett's rule.

When at least one of R^{1c} and R^{1h} represents a hydrogen atom or a substituent having a positive σp value of the Hammett's rule, an electron of the triazine ring is withdrawn.

In the case of representing a substituent having a positive σp value of the Hammett's rule, LUMO is stabilized due to the electron-withdrawing property of the substituent and this is preferred because the excitation life becomes short and the light resistance is enhanced.

The σp value of the substituent having a positive σp value of the Hammett's rule is preferably from 0.1 to 1.2, more preferably from 0.2 to 1.0, still more preferably from 0.4 to 0.8.

Preferred substituents having a positive σp value of the Hammett's rule include COOR^{r}, CONR^{s}₂, a cyano group, a trifluoromethyl group, a halogen atom, a nitro group, NHSO₂CH₃, and SO₃M. Among these, a cyano group, a nitro group and NHSO₂CH₃ are preferred. Here, each of R^{r} and R^{s} represents a hydrogen atom or a monovalent substituent, and M represents a hydrogen atom or an alkali metal.

R¹ⁿ is, in view of raising the gram extinction coefficient, preferably a hydrogen atom, an alkoxy group (an alkoxy group having a carbon number of 1 to 20, preferably an alkoxy group having a carbon number of 1 to 10, more preferably a methoxy group or an ethoxy group, still more preferably a methoxy group), an amino group (preferably a substituted amino group, more preferably a dimethylamino group or a diethylamino group), an alkyl group (preferably an alkyl group having a carbon number of 1 to 20, more preferably an alkyl group having a carbon number of 1 to 10, still more preferably an alkyl group substituted with a fluorine atom, yet still more preferably a trifluoromethyl group), a sulfonamido group or an aryl group (preferably an aryl group having a carbon number of 6 to 20, e.g., phenyl group, naphthyl group), more preferably a hydrogen atom or an alkoxy group, still more preferably an alkoxy group.

In formula (1), Y₁ represents -COR^{1q} or -SO₂R^{1r}, and each of R^{1q} and R^{1r} independently represents a monovalent substituent.

The monovalent substituent represented by R^{1q} and R^{1r} includes a substituent selected from Substituent Group A.

R^{1q} is, in view of raising the light resistance, preferably an alkyl group (preferably an alkyl group having a carbon number of 1 to 20, more preferably an alkyl group having a carbon number of 1 to 10, still more preferably a methyl group or a 1-ethylhexyl group), a perfluoroalkyl group (preferably a perfluoroalkyl group having a carbon number of 1 to 20, more preferably a perfluoroalkyl group having a carbon number of 1 to 10, still more preferably a trifluoromethyl group), a substituted or unsubstituted amino group (e.g., amino group, dimethylamino group, aniline group, substituted sulfoamino group; preferably an unsubstituted amino group), an alkoxy group (an alkoxy group having a carbon number of 1 to 20, preferably an alkoxy group having a carbon number of 1 to 10, more preferably a methoxy group or an ethoxy group, still more preferably a methoxy group) or an aryl group (preferably an aryl group having a carbon number of 6 to 20, e:g., phenyl group, naphthyl group), more preferably an alkyl group or a perfluoroalkyl group, still more preferably a perfluoroalkyl group.

R^{1r} is, in view of raising the light resistance, preferably an alkyl group (preferably an alkyl group having a carbon number of 1 to 20, more preferably an alkyl group having a carbon number of 1 to 10, still more preferably a methyl group or a 3-octyl group), a perfluoroalkyl group (preferably a perfluoroalkyl group having a carbon number of 1 to 20, more preferably a perfluoroalkyl group having a carbon number of 1 to 10, still more preferably a trifluoromethyl group) or an aryl group (preferably an aryl group having a carbon number of 6 to 20, more preferably an aryl group having a carbon number of 6 to 10, still more preferably a phenyl group or a naphthyl group, yet still more preferably a phenyl group), more preferably an alkyl group or a perfluoroalkyl group.

In formula (1), Y₁ preferably represents -SO₂R^{1r}, because the compound exhibits a high ultraviolet-blocking effect in the short wavelength ultraviolet region in the vicinity of 300 to 320 nm and enhances the light resistance. In the case of -SO₂R^{1r}, HOMO is more stabilized than in the case of -COR^{1q} and the absorption band on the long wavelength side out of two absorption bands characteristic of a triazine-based ultraviolet absorber can be more shifted to the short wavelength side, as a result, the compound can exhibit a high ultraviolet-blocking effect in the short wavelength ultraviolet region in the vicinity of 300 to 320 nm and be reduced in tinting. Also, the oxidation potential decreases and the light resistance is enhanced.

In formula (1), each of R^{1a}, R^{1e}, R^{1f} and R^{1j} independently represents a hydrogen atom or a monovalent substituent excluding -NHY₁ and -OH, Y₁ represents -COR^{1q} or -SO₂R^{1r}, and each of R^{1q} and R^{1r} independently represents a monovalent substituent.

In the case where each of R^{1a}, R^{1e}, R^{1f} and R^{1j} represents a monovalent substituent, the monovalent substituent includes a substituent selected from Substituent Group A.

Each of R^{1a}, R^{1e} and R^{1j} is preferably a hydrogen atom, because the absorption is steeply skirted (that is, the absorption end is sharp).

R^{1f} is preferably a hydrogen atom, an acyloxy group (preferably an acyloxy group having a carbon number of 2 to 10, e.g., acetoxy group), a formyl group, an arylthio group (preferably a phenylthio group) or a halogen atom, and a hydrogen atom is preferred because the absorption is steeply skirted (that is, the absorption end is sharp).

The compound represented by formula (1) is preferably a compound represented by the following formula (2):

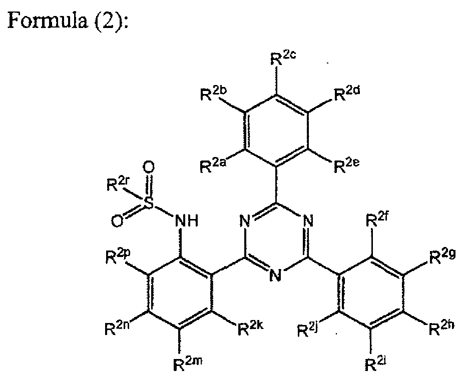

R^{2b}, R^{2c} R^{2d}, R^{2g}, R^{2h}, R²ⁱ, R^{2k}, R^{2m} , R²ⁿ and R^{2p} have the same meaning as R^{1b}, R^{1c}, R^{1d}, R^{1g}, R^{1h}, R¹ⁱ, R^{1k}, R^{1m}, R¹ⁿ and R^{1p} in formula (1), respectively; R^{2a}, R^{2e}, R^{2f} and R^{2j} have the same meanings as R^{1a}, R^{1e}, R^{1f} and R^{1j} in formula (1), respectively; and R^{2r} have the same meaning as R^{1r} in formula (1).

Specific examples and preferred examples of R^{2b}, R^{2c}, R^{2d}, R^{2g}, R^{2h}, R²ⁱ, R^{2k}, R^{2m}, R²ⁿ and R^{2p} are the same as specific examples and preferred examples of R^{1b}, R^{1c}, R^{1d}, R^{1g}, R^{1h}, R¹ⁱ, R^{1k}, R^{1m}, R¹ⁿ and R^{1p} in formula (1), respectively. Specific examples and preferred examples of R^{2a}, R^{2e}, R^{2f} and R^{2j} are the same as specific examples and preferred examples of R^{1a}, R^{1e}, R^{1f} and R^{1j} in formula (1), respectively. Specific examples and preferred examples of R^{2r} are the same as specific examples and preferred examples of R^{1r} in formula (1).

Specific examples of the compound represented by formula (1) are illustrated below, but the present invention is not limited thereto.

In specific examples, Me indicates a methyl group, Et indicates an ethyl group, and Ph indicates a phenyl group.

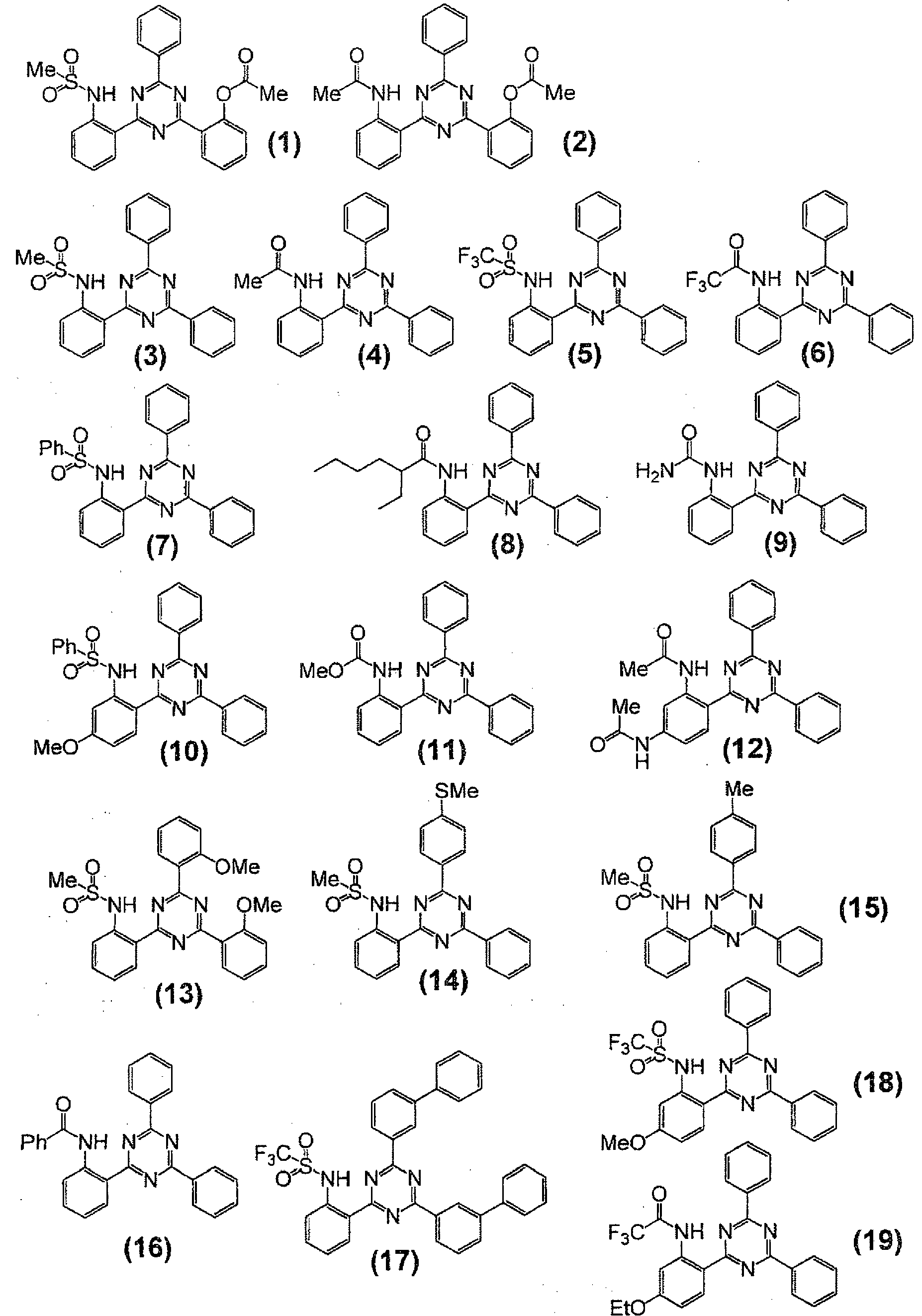

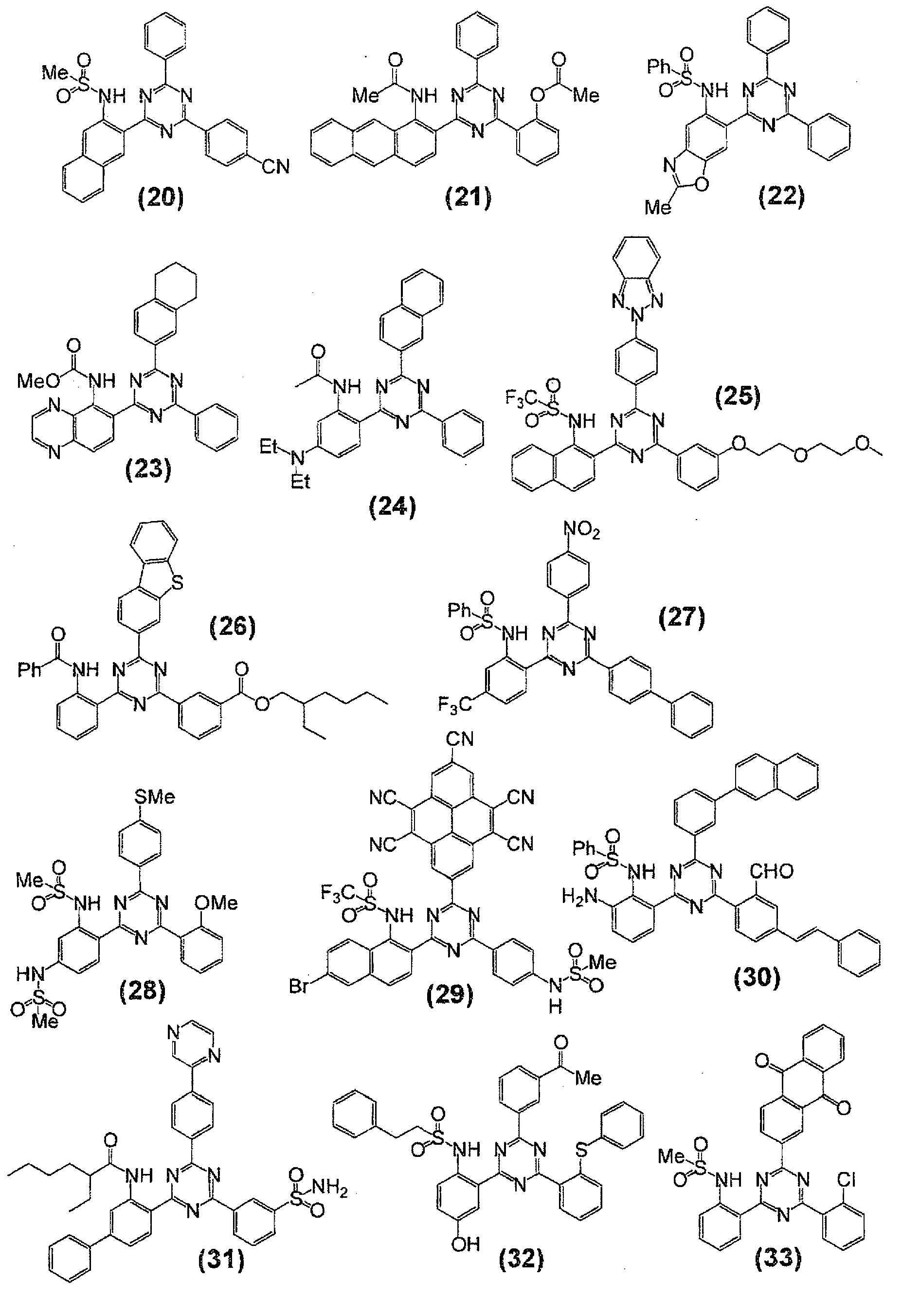

The compound represented by formula (1) may take a tautomer form depending on its structure and circumstances. In the present invention, the compound is described by referring to one representative form, but tautomers different from the description of the present invention are also included in the compound of the present invention.

The compound represented by formula (1) may contain an isotope (e.g., ²H, ³H, ¹³C, ¹⁵N, ¹⁷O, ¹⁸O).

The compound represented by formula (1) can be synthesized by an arbitrary method.

For example, the compound can be synthesized by referring to known patent documents or non-patent documents such as JP-A-7-188190, JP-A-11-315072, JP-A-2001-220385, Senryo to Yakuhin (Dyes and Chemicals), Vol, 40, No. 12, pp. 325-339 (1995), and J. Org. Chem., Vol. 27, pp. 3608-3613 (1962). Specifically, Exemplified Compound (3) can be synthesized by reacting anthranylamide, benzoyl chloride, methanesulfonyl chloride and benzamidine hydrochloride. This compound may be also synthesized by reacting 2-nitrobenzamidine hydrochloride, methyl benzimidate hydrochloride and methanesulfonyl chloride.

The compound represented by formula (1) can be suitably used as a light stabilizer, particularly, as an ultraviolet absorber.

The compound represented by formula (1) has an -NHY₁ group in a specific position of the 2,4,6-triphenyl-(1,3,5)triazine skeleton, and it is considered that the absorption peak on the long wavelength side out of two absorption peaks characteristic of triazine is shifted to the short wavelength side and two peaks are combined to produce an effect of exhibiting a blocking effect down to the short wavelength ultraviolet region near the wavelength of 300 to 320 nm and imparting excellent light resistance.

### [Ultraviolet Absorber]

The ultraviolet absorber represented by formula (1) is described blow.

The ultraviolet absorber of the present invention comprises the compound represented by formula (1).

In the ultraviolet absorber of the present invention, only one kind of a compound represented by formula (1) may be used, or two or more kinds of compounds may be used in combination.

The ultraviolet absorber of the present invention may be used in any form. Examples of the form include a liquid dispersion and a solution.

The maximum absorption wavelength of the ultraviolet absorber of the present invention is not particularly limited but is preferably from 290 to 330 nm, more preferably from 300 to 320 nm, and the half-value width is preferably from 40 to 120 nm, more preferably from 60 to 100 nm.

The maximum absorption wavelength and half-value width specified in the present invention can be easily measured by one skilled in the art. The measurement methods are described, for example, in Dai 4-Han Jikken Kagaku Koza 7 Bunko II 4th Edition, Experimental Chemistry Course 7, Spectroscopy II), pp. 180-186, edited by Chemical Society of Japan, Maruzen (1992). Specifically, these are determined by dissolving the sample in an appropriate solvent and measuring the solution in a spectrophotometer by using two quartz-made or glass-made cells, that is, one for the sample and another for control. The solvent used here is required, in combination with a property of dissolving the sample, for example, to have no absorption in the measurement wavelength region, exert less interaction with the solute molecule, and be not extremely volatile. An arbitrary solvent may be selected as long as it is a solvent satisfying the requirements above. In the present invention, the measurement is performed using ethyl acetate (EtOAc) as the solvent.

In the present invention, a value measured using ethyl acetate as the solvent and using a quartz cell with an optical path length of 10 mm is used for the maximum absorption wavelength and half-value width of the compound.

The half-value width of the spectrum is described, for example, in Dai 4-Han Jikken Kagaku Koza 3 Kihon Sosa III 4th Edition Experimental Chemistry Course 3, Basic Operation III), page 154, edited by Chemical Society of Japan, Maruzen (1991). Incidentally, the half-vale width is described in the literature above by labeling the abscissa with a wavenumber scale, but the half-value width used in the present invention is a value when the axis is marked with a wavelength scale, and the unit of the half-value width is nm. Specifically, the half-value width indicates the width of the absorption band of 1/2 of the absorbance at the maximum absorption wavelength and is used as a value indicative of the absorption spectral shape. A spectrum with a small half-value width is a sharp spectrum, and a spectrum with a large half-value width is a broad spectrum. The ultraviolet absorbing compound giving a broad spectrum has absorption also in a broad region on the longer wavelength side than the maximum absorption wavelength and therefore, in order to effectively block the light in the long wavelength ultraviolet region with no yellow tinting, an ultraviolet absorbing compound giving a spectrum with a small half-value width is preferred.

As described in Sumio Tokita, Kagaku Seminar 9, Color Chemistry (Chemistry Seminar 9, Color Chemistry), pp. 154-155, Maruzen (1982), the absorption intensity of light, namely, the oscillator intensity, is proportional to the integral of the molar extinction coefficient and when the absorption spectrum has good symmetry, the oscillator intensity is proportional to the product of the absorbance at the maximum absorption wavelength and the half-value width (here, the half-value width is a value when the axis is marked with a wavelength scale). This indicates that as long as the value of transition moment is the same, a compound giving a spectrum with a small half-value width exhibits large absorbance at the maximum absorption wavelength. Use of such an ultraviolet absorbing compound is advantageous in that light in the region around the maximum absorption wavelength can be effectively blocked only by its use in a small amount, but absorbance at a wavelength a little distance away from the maximum absorption wavelength rapidly decreases, and this makes it impossible to block light over a wide region.

The molar extinction coefficient at the maximum absorption wavelength of the ultraviolet absorber is preferably 20,000 or more, more preferably 30,000 or more, still more preferably 50,000 or more. With a molecular extinction coefficient of 20,000 or more, the absorption efficiency per mass of the ultraviolet absorber is sufficiently high, and the amount of the ultraviolet absorber used for completely absorbing light in the ultraviolet region can be reduced. This is preferred from the standpoint of preventing irritation to skin or accumulation in vivo and hardly causing bleed-out. Incidentally, the molar extinction coefficient used here is based on the definition described, for example, in Shin-han Jikken Kagaku Koza 9 Bunseki Kagaku [II] (New Edition. Experimental Chemistry Course 9, Analytical Chemistry [II]), page 244, edited by Chemical Society of Japan, Maruzen (1977) and can be determined together at the time of determining the above-described maximum absorption wavelength and half-value width.

The ultraviolet absorber of the present invention (hereinafter, sometimes simply referred to as an "ultraviolet absorber") may be also used in the state of a dispersion obtained by dispersing the ultraviolet absorber in a dispersion medium. An ultraviolet absorber dispersion containing the ultraviolet absorber of the present invention is described below.

The medium in which the ultraviolet absorber of the present invention is dispersed may be any medium. Examples thereof include water and an organic solvent. These may be used either singly or in combination.

Examples of the organic solvent as the dispersion medium used in the present invention include a hydrocarbon-based solvent such as pentane, hexane and octane, an aromatic solvent such as benzene, toluene and xylene, an ether-based solvent such as diethyl ether and methyl-tert-butyl ether, an alcohol-based solvent such as methanol, ethanol and isopropanol, an ester-based solvent such as acetone, ethyl acetate and butyl acetate, a ketone-based solvent such as methyl ethyl ketone, a nitrile-based solvent such as acetonitrile and propionitrile, an amide-based solvent such as N,N-dimethylformamide, N,N-dimethylacetamide and N-methylpyrrolidone, a sulfoxide-based solvent such as dimethylsulfoxide, an amine-based solvent such as triethylamine and tributylamine, a carboxylic acid-based solvent such as acetic acid and propionic acid, a halogen-based solvent such as methylene chloride and chloroform, and a heterocyclic solvent such as tetrahydrofuran and pyridine. These solvents may be also used in combination in an arbitrary ratio.

The dispersion may contain, together with the ultraviolet absorber, a dispersant, an antifoaming agent, a preservative, an anti-freezing agent, a surfactant and the like. Furthermore, the dispersion may contain arbitrary compounds in combination. Examples thereof include a dye, a pigment, an infrared absorber, a perfume, an inorganic material and a metal.

As the apparatus for obtaining a dispersion containing the ultraviolet absorber of the present invention, for example, a high-speed stirring type disperser having a large shear force or a disperser giving a high-strength ultrasonic energy may be used. Specific examples thereof include a colloid mill, a homogenizer, a capillary-type emulsifying device, a liquid siren, an electromagnetic distortion-type ultrasonic generator, and an emulsifying device with a Pohlmann whistle. The high-speed stirring-type disperser for use in the present invention is preferably a disperser of a type where the main part executing a dispersing action rotates at a high speed (from 500 to 15,000 rpm, preferably from 2,000 to 4,000 rpm) in a liquid, such as dissolver, polytron, homomixer, homoblender, keddy mill and jet agitator. The high-speed stirring-type disperser for use in the present invention is also called "dissolver" or "high-speed impeller disperser", and, as described in JP-A-55-129136, a disperser where a high-speed rotating shaft is equipped with an impeller obtained by folding a sawtooth plate alternately in the vertical direction is one preferred example.

An emulsified dispersion containing a hydrophobic compound may be prepared according to various processes. For example, in dissolving a hydrophobic compound in an organic solvent, the hydrophobic compound is dissolved in one kind of a solvent or a mixture of two or more kinds of solvents containing a plurality of arbitrary components, selected from a high-boiling-point organic material, a water-immiscible low-boiling-point organic solvent and a water-miscible organic solvent, and the solution is then dispersed in water or an aqueous hydrophilic colloid solution in the presence of a surfactant compound. The method for mixing a water-insoluble phase containing the hydrophobic compound and an aqueous phase may be a so-called forward mixing method of adding the water-insoluble phase in the aqueous phase under stirring or a back mixing method where the phases are reversed.

The ultraviolet absorber of the present invention may be also used in a solution state of being dissolved in a liquid medium. An ultraviolet absorber solution containing the ultraviolet absorber of the present invention is described below.

The liquid in which the ultraviolet absorber of the present invention is dissolved may be any liquid. Examples thereof include water and an organic solvent. The organic solvent includes those described above as a dispersion medium. These may be used either singly or in combination.

The solution of the ultraviolet absorber of the present invention may additionally contain arbitrary compounds in combination. Examples thereof include a dye, a pigment, an infrared absorber, a perfume, an inorganic material and a metal. Compounds other the ultraviolet absorber of the present invention may not be necessarily dissolved.

The content of the ultraviolet absorber in the solution containing the ultraviolet absorber of the present invention varies depending on the intended use and the form of usage and cannot be indiscriminately specified but may be an arbitrary concentration according to the intended use. The content is preferably from 0.001 to 30 mass%, more preferably from 0.01 to 10 mass%, based on the entire amount of the solution. Also, the solution may be previously prepared in a high concentration and used by diluting it when desired. The diluting solvent may be arbitrarily selected from the above-described solvents.

Examples of the material that is stabilized by the ultraviolet absorber of the present invention include a dye, a pigment, food, a beverage, a health care product, a vitamin preparation, medicine, ink, oil, fat, wax, a surface coating, a cosmetic material, a photographic material, a fabric and a dye therefor, and a coating material.

In the case of using the ultraviolet absorber of the present invention, the mode thereof may be any method. The ultraviolet absorber of the present invention may be used by itself or as a composition but is preferably used as a composition.

### [Examples]

The present invention is described in greater detail below, but the present invention is not limited thereto.

### (Synthesis of Exemplified Compound (1))

To 100 g of anthranylic acid, 1,000 mL of an aqueous saturated sodium hydrogencarbonate solution was added and to this aqueous solution, 85 mL of methanesulfonyl chloride was added dropwise at 0°C, followed by stirring at 0°C for 2 hours. To the resulting reaction solution, 50 mL of hydrochloric acid (35 mass%) was added, and the obtained solid was filtered and washed with water to obtain 142 g of Synthesis Intermediate A (yield: 90%).

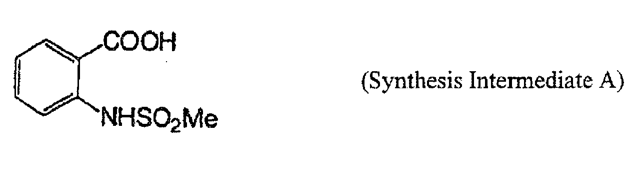

Thereafter, 40 g of Synthesis Intermediate A, 26 g of salicylamide and 2 mL of DMF (N,N-dimethylformamide) were added to 800 mL of toluene, and 22 g of thionyl chloride was added dropwise at room temperature. This solution was stirred at 85°C for 2 hours, and 3.9 g of p-toluenesulfonic acid monohydrate was added, followed by stirring at 130°C for 5 hours. The resulting reaction solution was cooled to 60°C and after adding 30 mL of triethylamine, cooled to room temperature. To this solution, 300 mL of methanol was added, and the obtained solid was filtered and washed with methanol to obtain 52 g of Synthesis Intermediate B (yield: 88%).

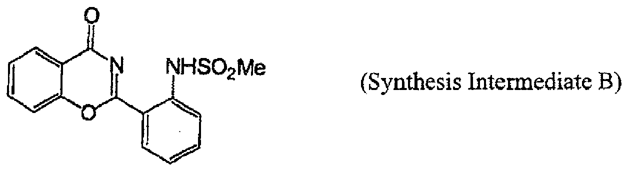

Separately, 1,000 mL of methanol and 30 g of a 28% sodium methoxide methanol solution were added to 25 g of benzamidine hydrochloride. To this solution, 45 g of Synthetic Intermediate B was added, and after stirring at room temperature for 3 hours, 1 mL of 35 mass% hydrochloric acid was added. The obtained solid was filtered and washed with water and methanol to obtain 11 g of Synthesis Intermediate C (yield: 92%).

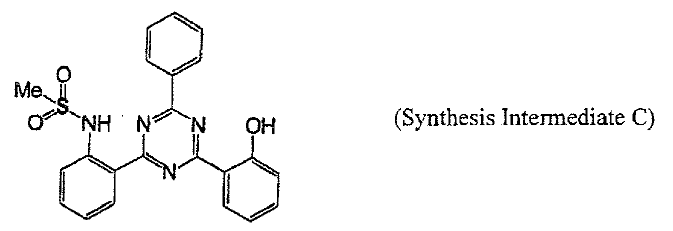

To 2.0 g of Synthesis Intermediate C, 50 mL of pyridine and 0.5 g of acetic anhydride were added, and this mixture stirred at room temperature for 5 hours. The resulting reaction solution was concentrated in an evaporator and then dispersed in water, and the obtained solid was filtered and washed with water to obtain 1.9 g of Exemplified Compound (1) (yield: 86%).

### (Synthesis of Exemplified Compound (3))

In 300 mL of dimethylacetamide, 100 g of anthranylamide was dissolved, and 103 g of benzoyl chloride was added dropwise at 0°C. This solution was stirred at room temperature for 3 hours, and the obtained solid was filtered and washed with water and acetone to obtain 161 g of a solid. To 50 g of this solid, 2,000 mL of methanol was added, and an aqueous solution prepared by dissolving 68 g of cesium carbonate in 300 mL of water was added dropwise. This mixture was stirred under reflux for 2 hours and after cooling to room temperature, 40 mL of 35 mass% hydrochloric acid was added dropwise. Furthermore, 400 mL of water was added, and the obtained solid was filtered and washed with water to obtain 33 g of Synthesis Intermediate D.

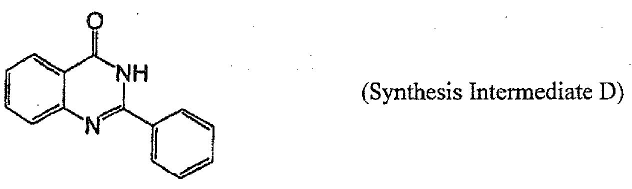

To 5.0 g of Synthesis Intermediate D, 200 mL of dimethylformamide and 23 g of triethylamine were added, and 2.6 g of methanesulfonyl chloride was added dropwise at 0°C. To this reaction solution, 20 mL of water was added, and the obtained solid was filtered and washed with water to obtain 6.0 g of Synthesis Intermediate E.

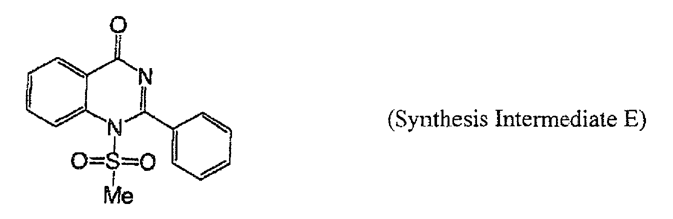

To 5.0 g of benzamidine hydrochloride, 1,000 mL of methanol and 6.0 g of a 28% sodium methoxide methanol solution were added, and 10.0 g of Synthetic Intermediate E was added at room temperature. A reaction was allowed to proceed at 60°C for 5 hours and after cooling to room temperature, 0.5 mL of 35 mass% hydrochloric acid was added. The obtained solid was washed with water and methanol to obtain Exemplified Compound (3).

### (Synthesis of Exemplified Compound (4))

Exemplified Compound (4) was synthesized by the same synthesis method except for using acetyl chloride in place of methanesulfonyl chloride in the step of obtaining Synthesis Intermediate E in the synthesis of Exemplified Compound (3).

### (Synthesis of Exemplified Compound (5))

Exemplified Compound (5) was synthesized by the same synthesis method except for using trifluoromethanesulfonyl chloride in place of methanesulfonyl chloride in the step of obtaining Synthesis Intermediate E in the synthesis of Exemplified Compound (3).

### (Synthesis of Exemplified Compound (6))

Exemplified Compound (6) was synthesized by the same synthesis method except for using trifluoroacetyl chloride in place of methanesulfonyl chloride in the step of obtaining Synthesis Intermediate E in the synthesis of Exemplified Compound (3).

### (Synthesis of Exemplified Compound (7))

Exemplified Compound (7) was synthesized by the same synthesis method except for using benzenesulfonyl chloride in place of methanesulfonyl chloride in the step of obtaining Synthesis Intermediate E in the synthesis of Exemplified Compound (3).

### (Synthesis of Exemplified Compound (8))

Exemplified Compound (8) was synthesized by the same synthesis method except for using 2-ethylhexanoyl chloride in place of methanesulfonyl chloride in the step of obtaining Synthesis Intermediate E in the synthesis of Exemplified Compound (3).

### (Synthesis of Exemplified Compound (13))

Exemplified Compound (13) was synthesized by the same synthesis method except for using 2-methoxybenzoyl chloride in place of benzoyl chloride in the step of obtaining Synthesis Intermediate D in the synthesis of Exemplified Compound (3) and using 3-methoxybenzamidine hydrochloride in place of benzamidine hydrochloride in the final step.

### (Synthesis of Exemplified Compound (16))

Exemplified Compound (16) was synthesized by the same synthesis method except for using benzoyl chloride in place of methanesulfonyl chloride in the step of obtaining Synthesis Intermediate E in the synthesis of Exemplified Compound (3).

### (Synthesis of Exemplified Compound (17))

Exemplified Compound (17) was synthesized by the same synthesis method except for using 3-phenylbenzoyl chloride in place of benzoyl chloride in the step of obtaining Synthesis Intermediate D in the synthesis of Exemplified Compound (3) and using 3-phenylbenzamidine hydrochloride in place of benzamidine hydrochloride in the final step.

### (Synthesis of Exemplified Compound (18))

Exemplified Compound (18) was synthesized by the same synthesis method except for using 4-methoxyanthranylamide in place of anthranylamide as raw material in the synthesis of Exemplified Compound (5).

### (Synthesis of Exemplified Compound (19))

Exemplified Compound (19) was synthesized by the same synthesis method except for using 4-ethoxyanthranylamide in place of anthranylamide as raw material in the synthesis of Exemplified Compound (6).

### (Synthesis of Exemplified Compound (24))

To 18.4 g of cyanuric chloride, 200 mL of o-dichlorobenzene was added, and 35.0 g of aluminum chloride was added thereto, followed by stirring at room temperature for 1 hour. The resulting reaction solution was cooled to 0°C and after adding 12.1 g of benzene and 13.1 g of naphthalene, stirred for 2 hours. The solution was further stirred at room temperature for 2 hours, and 20.6 g of N-[3-(diethylamino)phenyl]acetamide was added, followed by stirring at 60°C for 3 hours. After the reaction, water was added, and the reaction solution was extracted with dichloromethane and concentrated in an evaporator to synthesize Exemplified Compound (24).

### (Synthesis of Exemplified Compound (28))

To 18.4 g of cyanuric chloride, 200 mL of o-dichlorobenzene was added, and 35.0 g of aluminum chloride was added thereto, followed by stirring at room temperature for 1 hour. The resulting reaction solution was cooled to 0°C and after adding 12.4 g of (methylsulfanyl)benzene, stirred for 2 hours. Furthermore, 10.8 g of methoxybenzene was added, and the mixture was stirred for 2 hours. This solution was further stirred at room temperature for 3 hours, and 26.4 g of N-{3-[(methylsulfonyl)amino]phenyl}methanesulfonamide was added, followed by stirring at 100°C for 3 hours. After the reaction, water was added, and the reaction solution was extracted with dichloromethane and concentrated in an evaporator to obtain Exemplified Compound (28).

### (Synthesis of Exemplified Compound (33))

Exemplified Compound (33) was synthesized by the same synthesis method except for using anthraquinone-2-carbonyl chloride in place of benzoyl chloride as raw material and using 2-chloro-benzamidine hydrochloride in place of benzamidine hydrochloride in the synthesis of Exemplified Compound (3).

### (Evaluation of Light Resistance)

A sample solution (Sample No. 1) was prepared by dissolving 1 mg of Exemplified Compound (1) in 100 ml of ethyl acetate. Sample solutions (Sample Nos. 2 to 23) were synthesized in the same manner by using Exemplified Compound (3), Exemplified Compound (4), Exemplified Compound (5), Exemplified Compound (6), Exemplified Compound (7), Exemplified Compound (8), Exemplified Compound (13), Exemplified Compound (16), Exemplified Compound (17), Exemplified Compound (18), Exemplified Compound (19), Exemplified Compound (20), Exemplified Compound (22), Exemplified Compound (24), Exemplified Compound (25), Exemplified Compound (27), Exemplified Compound (28), Exemplified Compound (29), Exemplified Compound (33), Comparative Compound (1) ("TINUVIN 1577" produced by Ciba Specialty Chemicals), Comparative Compound (2) ("UVINUL 3035" produced by BASF), and Comparative Compound (3).

Each sample solution was measured for the absorbance at 250 to 450 nm by using a spectrophotometer, UV-3600 (trade name), manufactured by Shimadzu Corporation with a quartz cell having an optical path length of 10 mm.

The cell enclosing the sample solution was irradiated with light by using a metal halide lamp (EYE SUPER UV Tester, trade name, manufactured by Iwasaki Electric Co., Ltd.) under the conditions of an illuminance of 900 W/m², a temperature of 63°C and a humidity of 50%, and the residual amount of each compound was measured after 30 hours and after 60 hours. The residual amount was calculated according to the following formula.

Residual amount (%) = 100 x (absorbance after irradiation)/(absorbance before irradiation)

Here, the absorbance is a value measured at the maximum absorption wavelength of each compound. The results are shown in Table 1.

A larger residual amount indicates higher light resistance.

**Table 1**

| Sample No. | Compound | Residual Amount After 30 Hours (%) | Residual Amount After 60 Hours (%) | Remarks |
|---|---|---|---|---|
| 1 | Exemplified Compound (1) | 99 | 85 | Invention |
| 2 | Exemplified Compound (3) | 99 | 94 | Invention |
| 3 | Exemplified Compound (4) | 99 | 76 | Invention |
| 4 | Exemplified Compound (5) | 99 | 99 | Invention |
| 5 | Exemplified Compound (6) | 99 | 82 | Invention |
| 6 | Exemplified Compound (7) | 99 | 95 | Invention |
| 7 | Exemplified Compound (8) | 99 | 76 | Invention |
| 8 | Exemplified Compound (13) | 99 | 84 | Invention |
| 9 | Exemplified Compound (16) | 99 | 73 | Invention |
| 10 | Exemplified Compound (17) | 99 | 96 | Invention |
| 11 | Exemplified Compound (18) | 99 | 97 | Invention |
| 12 | Exemplified Compound (19) | 99 | 75 | Invention |
| 13 | Exemplified Compound (20) | 99 | 99 | Invention |
| 14 | Exemplified Compound (22) | 99 | 99 | Invention |
| 15 | Exemplified Compound (24) | 99 | 87 | Invention |
| 16 | Exemplified Compound (25) | 99 | 95 | Invention |
| 17 | Exemplified Compound (27) | 99 | 99 | Invention |
| 18 | Exemplified Compound (28) | 99 | 94 | Invention |
| 19 | Exemplified Compound (29) | 99 | 99 | Invention |
| 20 | Exemplified Compound (33) | 99 | 99 | Invention |
| 21 | Comparative Compound (1) | 51 | 27 | Comparative Example |
| 22 | Comparative Compound (2) | 2 | 1 | Comparative Example |
| 23 | Comparative Compound (3) | 64 | 35 | Comparative Example |

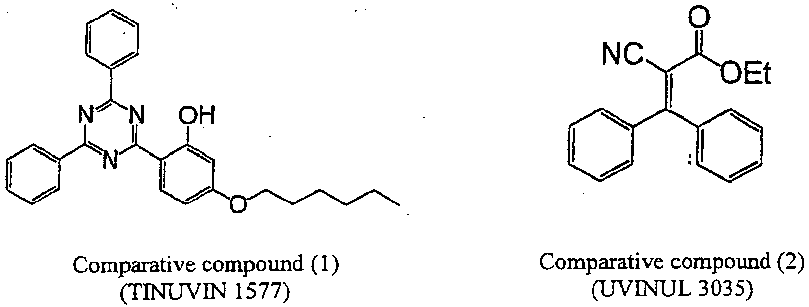

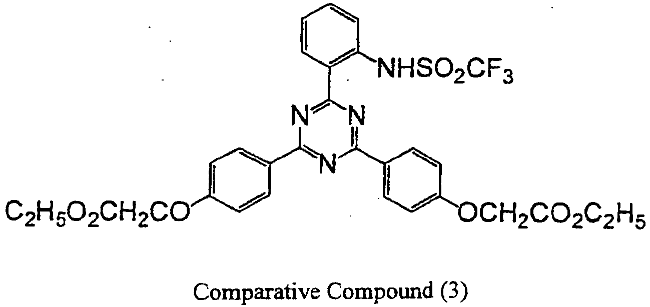

## Claims

1. A compound represented by the following formula (1):
Formula (I): 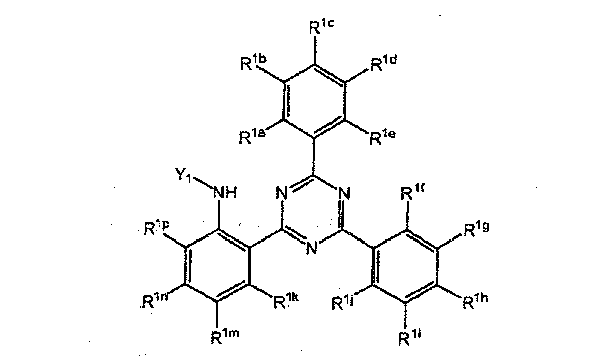 wherein each of R^{1b}, R^{1c}, R^{1d}, R^{1g}, R^{1h}, R¹ⁱ, R^{1k}, R^{1m}, R¹ⁿ and R^{1p} independently represents a hydrogen atom or a monovalent substituent, the monovalent substituents may combine with each other to form a ring, provided that at least one of R^{1c} and R^{1h} represents a hydrogen atom or a substituent having a positive σp value of the Hammett's rule, each of R^{1a}, R^{1e}, R^{1f} and R^{1j} independently represents a hydrogen atom or a monovalent substituent excluding -NHY₁ and -OH, Y₁ represents -COR^{1q} or -SO₂R^{1r} and each of R^{1q} and R^{1r} independently represents a monovalent substituent.

2. The compound as claimed in claim 1, wherein the compound represented by formula (1) is a compound represented by the following formula (2):
Formula (2): 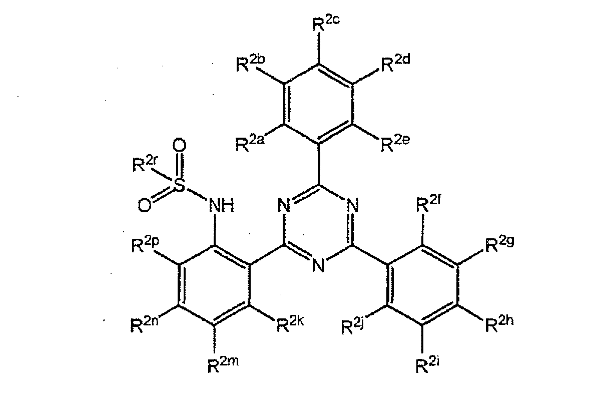 wherein R^{2b}, R^{2c}, R^{2d}, R^{2g}, R^{2h}, R²ⁱ, R^{2k}, R^{2m}, R²ⁿ and R^{2p} have the same meanings as R^{1b}, R^{1c}, R^{1d}, R^{1g} R^{1h}, R¹ⁱ, R^{1k}, R^{1m} R¹ⁿ and R^{1p} in formula (1), respectively; R^{2a}, R^{2e}, R^{2f} and R^{2j} have the same meanings as R^{1a}, R^{1e}, R^{1f} and R^{1j} in formula (1), respectively; and R^{2r} have the same meaning as R^{1r} in formula (1).

3. The compound as claimed in claim 1, wherein R^{1q} represents an alkyl group, a perfluoroalkyl group, an amino group, an aryl group or an alkoxy group.

4. The compound as claimed in claim 1, wherein R^{1r} represents an alkyl group, a perfluoroalkyl group or an aryl group.

5. The compound as claimed in claim 1 or 3, wherein R^{1q} represents an alkyl group or a perfluoroalkyl group.

6. The compound as claimed in claim 1 or 4, wherein R^{1r} represents an alkyl group or a perfluoroalkyl group.

7. The compound as claimed in any one of claims 1 and 3 to 6, wherein R^{1c} is a hydrogen atom, an alkyl group, an alkoxy group, an alkylthio group, an aryl group, a heterocyclic group or a nitro group.

8. The compound as claimed in any one of claims 1 and 3 to 7, wherein R¹ⁿ is a hydrogen atom, an alkoxy group, an amino group, an alkyl group, a sulfonamido group or an aryl group.

9. The compound as claimed in any one of claims 1 and 3 to 7, wherein R¹ⁿ is a hydrogen atom or an alkoxy group.

10. The compound as claimed in any one of claims 1 and 3 to 9, wherein each of R^{1b}, R^{1d}, R^{1g}, R^{1h}, R¹ⁱ, R^{1k}, R^{1m} and R^{1p} is independently a hydrogen atom, an alkoxy group, an aryl group, a cyano group, an alkoxycarbonyl group, an acylamido group, an alkenyl group, a sulfamoyl group, an acyl group or a hydroxy group.

11. The compound as claimed in any one of claims 1 and 3 to 9, wherein each of R^{1b}, R^{1d}, R^{1g}, R^{1h}, R¹ⁱ, R^{1k}, R^{1m} and R^{1p} is a hydrogen atom.

12. The compound as claimed in any one of claims 1 and 3 to 11, wherein each of R^{1a}, R^{1e} and R^{1j} is a hydrogen atom.

13. The compound as claimed in any one of claims 1 and 3 to 12, wherein R^{1f} is a hydrogen atom, an acyloxy group, a formyl group, an arylthio group or a halogen atom.

14. The compound as claimed in any one of claims 1 and 3 to 6, wherein at least two of R^{1b}, R^{1c} and R^{1d}, a pair of R^{1h} and R¹ⁱ, or at least two of R^{1m}, R¹ⁿ and R^{1p} are combined to form a ring.

15. An ultraviolet absorber comprising the compound claimed in any one of claims 1 to 14.

## Patentansprüche

1. Verbindung, dargestellt durch die folgende Formel (1):
Formel (1) : 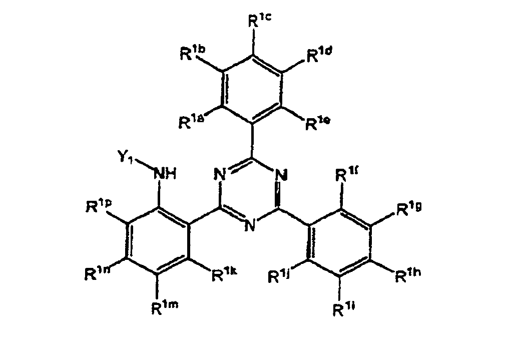 worin jedes von R^{1b}, R^{1c}, R^{1d}, R^{1g}, R^{1h}, R¹ⁱ, R^{1k}, R^{1m}, R¹ⁿ und R^{1p} unabhängig ein Wasserstoffatom oder einen monovalenten Substituenten darstellt, wobei die monovalenten Substituenten miteinander verbunden sein können, um einen Ring zu bilden, vorausgesetzt, dass mindestens eines von R^{1c} und R^{1h} ein Wasserstoffatom oder einen Substituenten, der einen positiven σb-Wert der Hammett-Regel aufweist, darstellt, jedes von R^{1a}, R^{1e}, R^{1f} und R^{1j} unabhängig ein Wasserstoffatom oder einen monovalenten Substituenten, mit Ausnahme von -NHY₁ und -OH, darstellt, Y, -COR^{1q} oder -SO₂R^{1r} darstellt und jedes von R^{1q} und R^{1r} unabhängig einen monovalenten Substituenten darstellt.

2. Verbindung gemäß Anspruch 1, worin die durch die Formel (1) dargestellte Verbindung eine durch die folgende Formel (2) dargestellte Verbindung ist:
Formel (2): 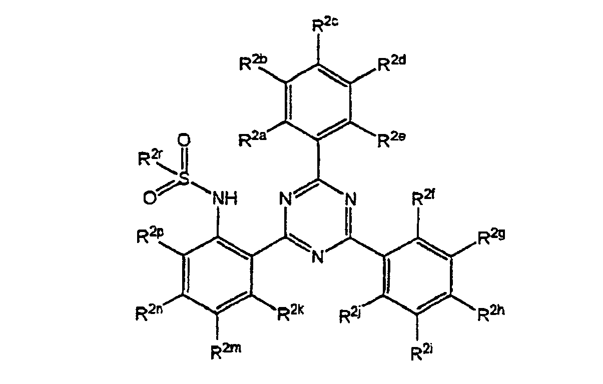 worin R^{2b}, R^{2c}, R^{2d}, R^{2g}, R^{2h}, R²ⁱ, R^{2k}, R^{2m}, R²ⁿ und R^{2p} die gleichen Bedeutungen wie R^{1b}, R^{1c}, R^{1d}, R^{1g}, R^{1h}, R¹ⁱ, R^{1k}, R^{1m}, R¹ⁿ bzw. R^{1p} in Formel (1) besitzen, R^{2a}, R^{2e}, R^{2f} und R^{2j} die gleichen Bedeutungen wie R^{1a}, R^{1e}, R^{1f} bzw. R^{1j} in Formel (1) besitzen; und R^{2r} die gleiche Bedeutung wie R^{1r} in Formel (1) besitzt.

3. Verbindung gemäß Anspruch 1, worin R^{1q} eine Alkylgruppe, eine Perfluoralkylgruppe, eine Aminogruppe, eine Arylgruppe oder eine Alkoxygruppe darstellt.

4. Verbindung gemäß Anspruch 1, worin R^{1r} eine Alkylgruppe, eine Perfluoralkylgruppe oder eine Arylgruppe darstellt.

5. Verbindung gemäß Anspruch 1 oder 3, worin R^{1q} eine Alkylgruppe oder eine Perfluoralkylgruppe darstellt.

6. Verbindung gemäß Anspruch 1 oder 4, worin R^{1r} eine Alkylgruppe oder eine Perfluoralkylgruppe darstellt.

7. Verbindung gemäß irgendeinem der Ansprüche 1 und 3 bis 6, worin R^{1c} ein Wasserstoffatom, eine Alkylgruppe, eine Alkoxygruppe, eine Alkylthiogruppe, eine Arylgruppe, eine heterocyclische Gruppe oder eine Nitrogruppe darstellt.

8. Verbindung gemäß irgendeinem der Ansprüche 1 und 3 bis 7, worin R¹ⁿ ein Wasserstoffatom, eine Alkoxygruppe, eine Aminogruppe, eine Alkylgruppe, eine Sulfonamidgruppe oder eine Arylgruppe darstellt.

9. Verbindung gemäß irgendeinem der Ansprüche 1 und 3 bis 7, worin R¹ⁿ ein Wasserstoffatom oder eine Alkoxygruppe ist.

10. Verbindung gemäß irgendeinem der Ansprüche 1 und 3 bis 9, worin jedes von R^{1b}, R^{1d}, R^{1g}, R^{1h}, R¹ⁱ, R^{1k}, R^{1m} und R^{1p} unabhängig ein Wasserstoffatom, eine Alkoxygruppe, eine Arylgruppe, eine Cyanogruppe, eine Alkoxycarbonylgruppe, eine Acylamidogruppe, eine Alkenylgruppe, eine Sulfamoylgruppe, eine Acylgruppe oder eine Hydroxygruppe darstellt.

11. Verbindung gemäß irgendeinem der Ansprüche 1 und 3 bis 9, worin jedes von R^{1b}, R^{1d}, R^{1g}, R^{1h}, R¹ⁱ, R^{1k}, R^{1m} und R^{1p} ein Wasserstoffatom ist.,

12. Verbindung gemäß irgendeinem der Ansprüche 1 und 3 bis 11, worin jede von R^{1a}, R^{1e} und R^{1j} ein Wasserstoffatom ist.

13. Verbindung gemäß irgendeinem der Ansprüche 1 und 3 bis 12, worin R^{1f} ein Wasserstoffatom, eine Acyloxygruppe, eine Formylgruppe, eine Arylthiogruppe oder ein Halogenatom ist.

14. Verbindung gemäß irgendeinem der Ansprüche 1 und 3 bis 6, worin mindestens zwei von R^{1b}, R^{1c} und R^{1d}, ein Paar von R^{1h} und R¹ⁱ, oder mindestens zwei von R^{1m}, R¹ⁿ und R^{1p} miteinander verbunden sind, um einen Ring zu bilden.

15. UV-Absorber, umfassend die Verbindung gemäß irgendeinem der Ansprüche 1 bis 14.

## Revendications

1. Composé représenté par la formule suivante (1) : 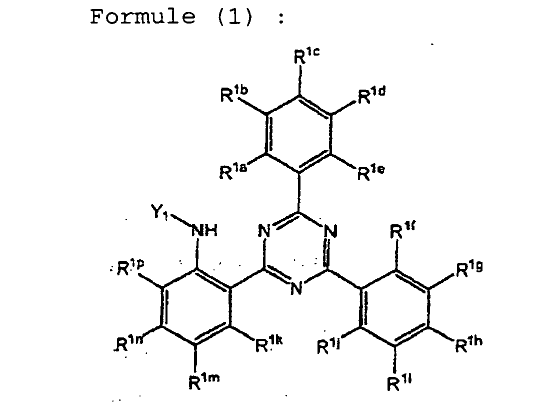 dans laquelle chacun de R^{1b}, R^{1c}, R^{1d}, R^{1g}, R¹ⁿ, R¹ⁱ, R^{1k}, R^{1m}, R¹ⁿ et R^{1p} représente indépendamment un atome d'hydrogène ou un substituant monovalent, les substituants monovalents peuvent se combiner l'un avec l'autre pour former un anneau, à condition qu'au moins un de R^{1c} et R^{1h} représente un atome d'hydrogène ou un substituant ayant une valeur σp positive de la règle de Hammett, chacun de R^{1a}, R^{1e}, R^{1f} et R^{1j} représente indépendamment un atome d'hydrogène ou un substituant monovalent excluant -NHY₁ et -OH, Y₁ représente -COR^{1q} ou : -SO₂R^{1r}, et chacun de R^{1q} et R^{1r} représente indépendamment un substituant monovalent.

2. Composé selon la revendication 1, dans lequel le composé représenté par la formule (1) est un composé représenté par la formule (2) suivante : 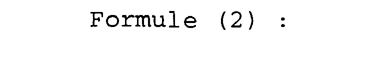 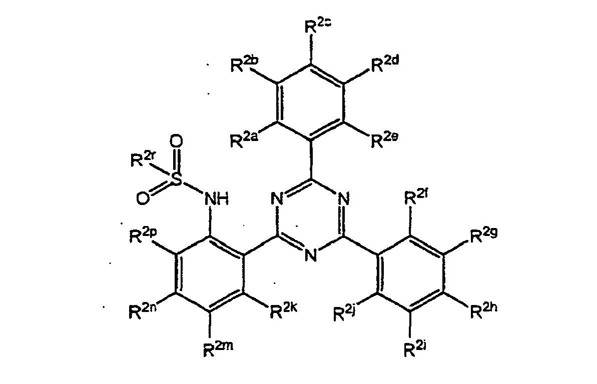 dans laquelle R^{2b}, R^{2c}, R^{2d}, R^{2g}, R^{2h}, R²ⁱ, R^{2k}, R^{2m}, R²ⁿ et R^{2p} ont respectivement les mêmes significations que R^{1b}, R^{1c}, R^{1a}, R^{1g}, R^{1h}, R¹ⁱ, R^{1k}, R^{1m}, R¹ⁿ et R^{1p} dans la formule (1), R^{2a}, R^{2e}, R^{2f} et R^{2j} ont respectivement les mêmes significations que R^{1a}, R^{1e}, R^{1f} et R^{1j} dans la formule (1) ; et R^{2r} a la même signification que R^{1r} dans la formule (1).

3. Composé selon la revendication 1, dans lequel R^{1q} représente un groupe alkyle, un groupe perfluoroalkyle, un groupe amino, un groupe aryle ou un groupe alcoxy.

4. Composé selon la revendication 1, dans lequel R^{1r} représente un groupe alkyle, un groupe perfluoroalkyle ou un groupe aryle.

5. Composé selon la revendication 1 ou 3, dans lequel R^{1q} représente un groupe alkyle ou un groupe perfluoroalkyle.

6. Composé selon la revendication 1 ou 4, dans lequel R^{1r} représente un groupe alkyle ou un groupe perfluoroalkyle.

7. Composé selon l'une quelconque des revendications 1 et 3 à 6, dans lequel R^{1c} est un atome d'hydrogène, un groupe alkyle, un groupe alcoxy, un groupe alkylthio, un groupe aryle, un groupe hétérocyclique ou un groupe nitro.

8. Composé selon l'une quelconque des revendications 1 et 3 à 7, dans lequel R¹ⁿ est un atome d'hydrogène, un groupe alcoxy, un groupe amino, un groupe alkyle, un groupe sulfonamido ou un groupe aryle.

9. Composé selon l'une quelconque des revendications 1 et 3 à 7, dans lequel R¹ⁿ est un atome d'hydrogène ou un groupe alcoxy.

10. Composé selon l'une quelconque des revendications 1 et 3 à 9, dans lequel chacun de R^{1b}, R^{1d}, R^{1g}, R^{1h}, R¹ⁱ, R^{1k}, R^{1m} et R^{1p} est indépendamment un atome d'hydrogène, un groupe alcoxy, un groupe aryle, un groupe cyano, un groupe alcoxycarbonyle, un groupe acylamido, un groupe alcényle, un groupe sulfamoyle, un groupe acyle ou un groupe hydroxy.

11. Composé selon l'une quelconque des revendications 1 et 3 à 9, dans lequel chacun de R^{1b}, R^{1d}, R^{1g}, R^{1h}, R¹ⁱ, R^{1k}, R^{1m} et R^{1p} est un atome d'hydrogène.

12. Composé selon l'une quelconque des revendications 1 et 3 à 11, dans lequel chacun de R^{1a}, R^{1e} et R^{1j} est un atome d'hydrogène.

13. Composé selon l'une quelconque des revendications 1 et 3 à 12, dans lequel R^{1f} est un atome d'hydrogène, un groupe acyloxy, un groupe formyle, un groupe arylthio ou un atome d'halogène.

14. Composé selon l'une quelconque des revendications 1 et 3 à 6, dans lequel au moins deux de R^{1b}, R^{1c} et R^{1d}, un couple de R^{1h} et R¹ⁱ, ou au moins deux de R^{1m}, R¹ⁿ et R^{1p} sont combinés pour former un anneau.

15. Absorbeur d'ultraviolet comprenant le composé selon l'une quelconque des revendications 1 à 14.
